Europäisches Patentamt

European Patent Office

Office européen des brevets

(11) Publication number: **0 329 358**
**A2**

# EUROPEAN PATENT APPLICATION

(21) Application number: **89301329.2**

(22) Date of filing: **13.02.89**

(51) Int. Cl.⁴: **A61M 5/315**

(30) Priority: **17.02.88 GB 8803655**

(43) Date of publication of application:
**23.08.89 Bulletin 89/34**

(84) Designated Contracting States:
**AT BE CH DE ES FR GB GR IT LI LU NL SE**

(71) Applicant: **R & R INVENTIONS LIMITED**
**24 Harborne Road Edgbaston**
**Birmingham B15 3AD(GB)**

(72) Inventor: **Power, Richard, Kiteley**
**Upper Woodston Farm Lindridge Tenbury**
**Wells**
**Hereford & Worcester WR15 8JQ(GB)**
Inventor: **Yair, John, David**
**23 Birmingham Road**
**Hagley Worcestershire DY9 9JZ(GB)**

(74) Representative: **Hands, Horace Geoffrey et al**
**GEORGE FUERY & CO Whitehall Chambers**
**23 Colmore Row**
**Birmingham B3 2BL(GB)**

(54) **Disposable syringe.**

(57) A disposable once only syringe for medical use has a tubular piston 22 connected to a piston rod 10 by a headed projection 18 20 passed through the piston bore, doubled over and then trapped in a recess 40 by a relative reverse movement of the parts. The parts are held together by the trapping of the projection in syringe filling, but upon ejection of contents the piston rod moves relative to the piston for a short distance to free the projection which allows the projection to disengage from the recess and move out of alignment with the recess. Any attempt to refill the syringe by a repeat operation causes the projection to pull through the piston and the piston rod to separate.

Fig. 1

## DISPOSABLE SYRINGE

This invention relates to disposable syringes for medical use and of the kind adapted for a single cycle of operations only. The object is to make it difficult or impossible for a syringe to be used on a succession of patients, in order to prevent the unintended transmission of disease from one to the next.

Many prior proposals for these purposes have suffered from the disadvantage that they require to be factory filled with the vaccine or like which is to be injected, and this creates difficulties due to different dosages being required as well as the possibility of different vaccines being needed for a succession of different patients. Other arrangements have been completely satisfactory from all aspects accept that they have been expensive to manufacture.

The object of the present invention is to solve these problems.

According to the invention a disposable syringe comprises a barrel, a piston and a piston-rod; in which the piston and rod are connected together by a projection on one of them extending through a bore in the other of them, said projection having a folded over end which is, in one working position, trapped and contained in an enlargement of said bore, and the projection and bore being of such lengths as to allow for lost motion of the one relative to the other, whereby the syringe can only effect a single operational cycle before the said end disengaging from the enlargement so as to allow the piston and rod to disengage from one another if a further operational cycle is attempted.

It will be appreciated that the barrel may be provided with an integral and attached needle or may be adapted for attachment of the needle.

The piston-rod may be of cruciform cross-section over the majority of its length and of similar dimension to the barrel so as to be guided for sliding movement in the (possibly) circular cross-section barrel. The rod may have an enlarged end for manual manipulation.

Two seals may be provided, namely a first one between the rod and piston effective when the piston and rod are in one position relative to one and another and a second effective in a second set of relative positions. The two positions are spaced by the lost motion movement.

One embodiment of the invention is now more particularly described with reference to the accompanying drawings wherein:-

Figure 1 is an enlarged scale fragmentary sectional view showing the piston being assembled to the piston rod in manufacture of the syringe;

Figures 2-4 show stages in assembly of the piston and piston rod together into the barrel in the manufacture of the syringe;

Figures 5-8 show stages in a cycle of operation of the assembled syringe.

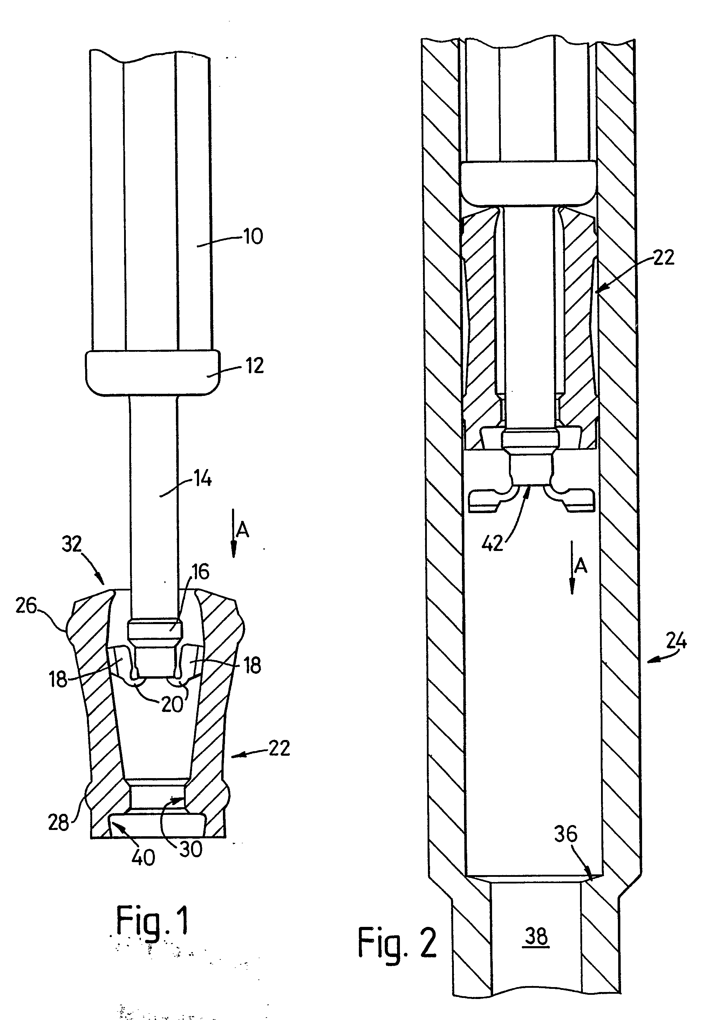

Turning to first to Figure 1, the piston rod is of conventional cruciform cross-section 10, and has a head 12 and an axially extending stem 14 provided with an annular boss 16 and, in this instance, a pair of parallel symmetrically located headed projections 18 at the free end of the stem 14.

The piston rod is for example moulded from a suitable synthetic resinous material. Each of the projections comprises a thin neck 20 which, because of the characteristics of the material of which it is made may be floppy or slightly springy.

The piston 22 is tubular, having an external diameter selected to be a close sliding fit in the barrel 24 and has peripheral ribs 26,28 at axially opposite ends for sealing with the barrel bore.

A piston bore includes an internal annular rib 30 dimensioned to be a close fit with the boss 16 on the piston rod extension, so as to provide a first piston -piston rod seal. The upper end of the piston adjacent the head 12 is of shallow frusto-conical shape at 32 so as to contact the head 12 at a narrow annular zone for the purpose of providing a second seal.

The piston bore is enlarged by a narrow angle frusto-conical recess 40 concentric to the piston axis opening with the smaller end of the frusto-cone at the free end of the piston, and located at the lower end of the piston as seen in the drawings. The piston is preferably moulded from a flexible resilient synthetic resinous material.

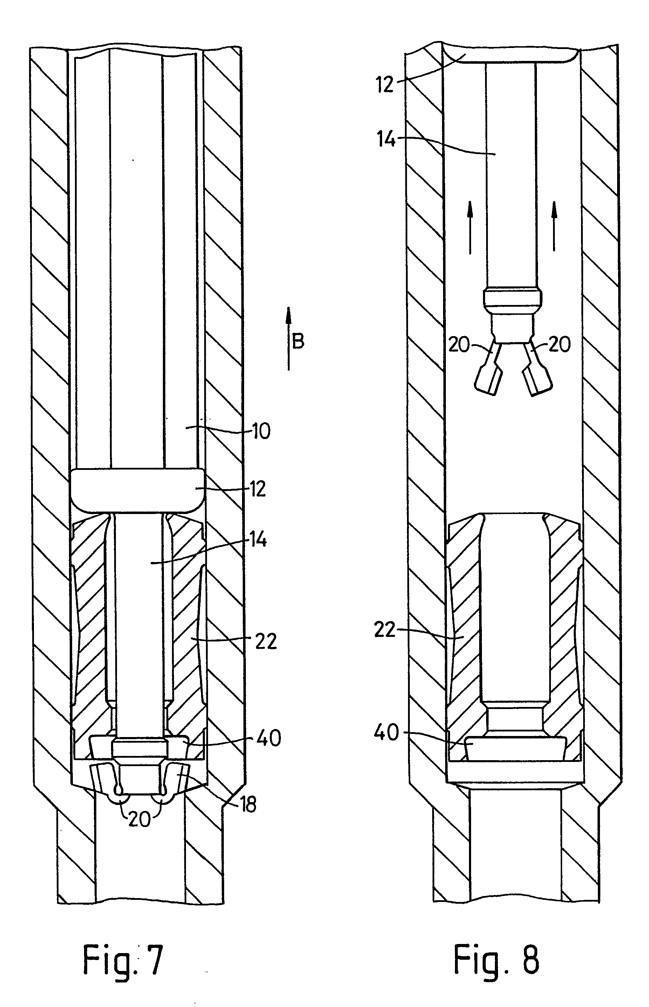

The piston and piston rod may be assembled together by arranging the two projections side by side, somewhat as in Figure 8, then pinching the two together and posting them through the piston bore. However, it has also been found that with the illustrated design due also to the flexibility of the piston material, that it is possible to force the rod and projections through the bore in the Figure 1 position with the projections folded back on themselves and located adjacent to the rod 14 between the rib 16 and the end of the rod 14, possibly with some distortion of the piston shape from a circular cross-section to an elliptical cross-section particularly at the upper end, which distortion is possible before the piston is located in the syringe barrel, and which distortion is reversed to restore the shape when the piston is inserted into the barrel.

The piston and rod are then assembled into the barrel as shown in Figure 2, when the projections, no longer constrained by the piston rod are free to diverge from the pinched together position or relax from the doubled back position, as the case may be. The movement of the piston rod relative to the piston in assembling together is shown by the arrow A in Figure 1, and the movement of the assembly relative to the barrel in insertion is shown by the like arrow A, Figure 2.

The barrel has an open end (not shown) at the insertion end and an internal shoulder 36 at the needle (not shown) end. The barrel portion 38 connected to the needle or for connection to the needle as the case may be, is of an internal dimension related to the piston rod and in particular to the spacing apart of the projections on the extension 14.

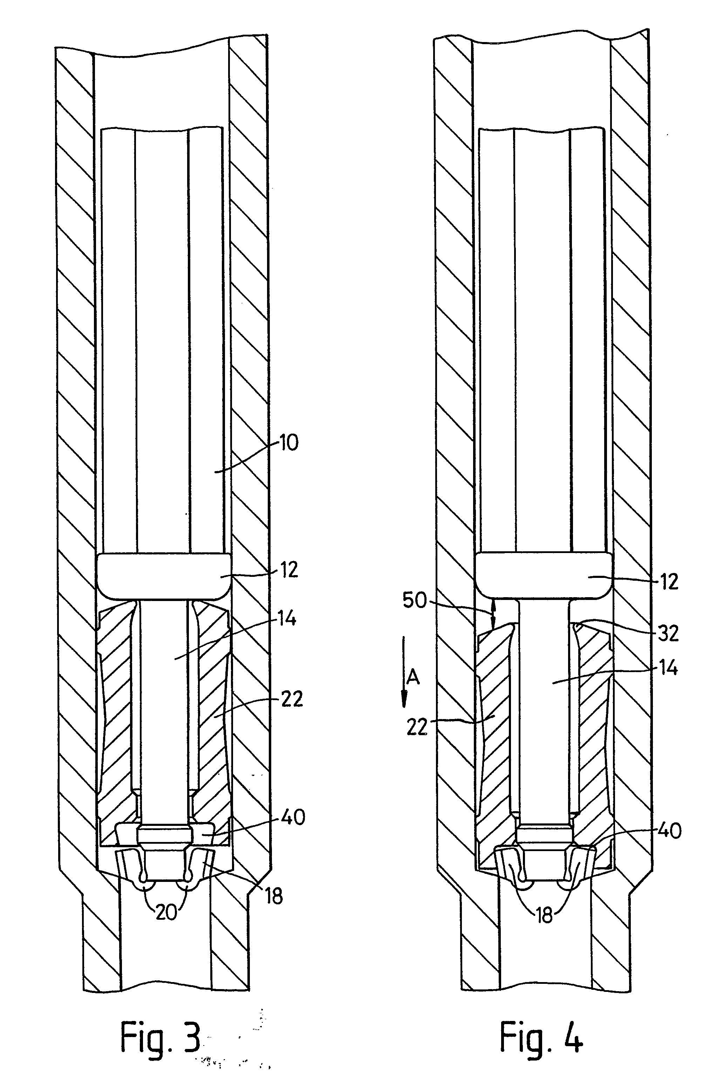

As the rod is displaced in the direction of arrow A, Figure 2, the projections 18 come to encounter the shoulder 36, due to the mentioned dimensions. Continued movement of the rod bends the projections back via the flexible necks, so that they lie closely against the rod between the end 42 of the same and the boss 16 Figure 3. The projections 18, 20 are dimensioned appropriately so that the projections will not extend over the annular boss in the doubled position of the projections. The transverse dimension of the doubled projections and rod 14 are then generally similar to that of the smaller end of the frusto-conical recess 40, without being so small as to be less than that of the normal (undistorted) minimum transverse dimension of the piston bore in the vicinity of the annular sealing rib.

When the parts are located in the Figure 3 position as the result of for example manual assembly, the piston is displaced, again in the direction of the arrow A, but relative to the piston rod. This can be accomplished in manufacture by a high pressure air blast directed into the syringe barrel from the upper and open end. The pressure acts on the upper end of the piston and displaces it relative to the piston rod until movement of the piston is terminated by the lower end of the same contacting the shoulder 36. This movement takes the projections 18 into the recess 40 where they are received and trapped. This movement also takes boss 16 into annular rib 30 to make the bottom seal and takes up the lost motion gap 50. The syringe is now ready for delivery, sale and use.

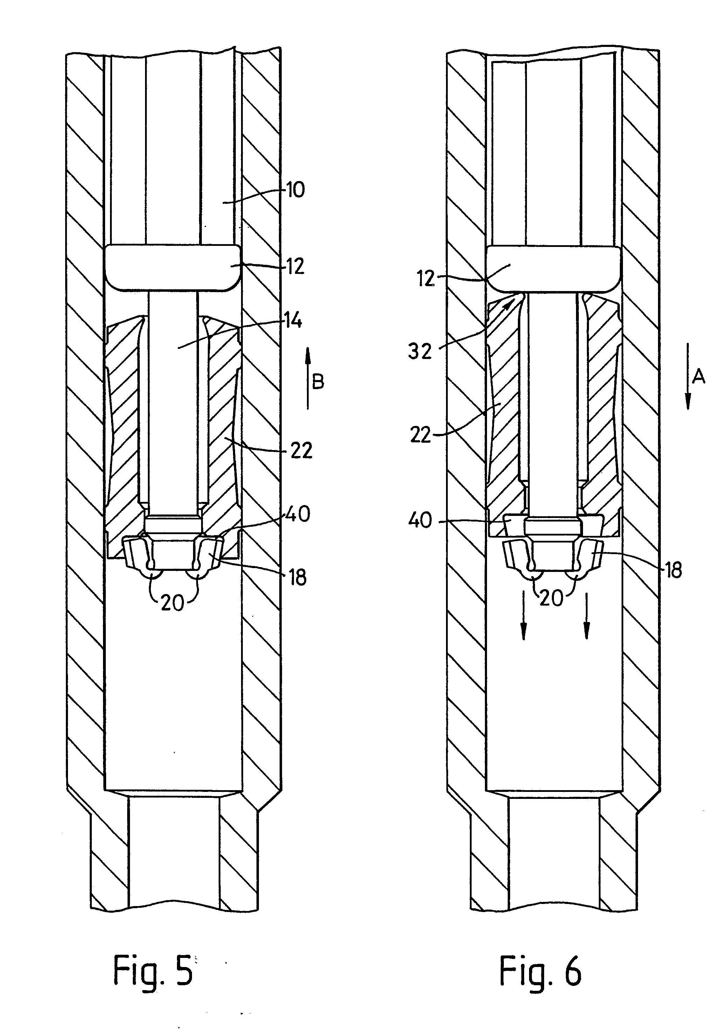

When the syringe is to be filled, the action for the user is purely conventional. The needle is inserted into the fluid source for example an ampoule of vaccine in the usual way, and the piston rod is withdrawn relative to the barrel, that is displaced in the direction of arrow B Figure 5. The bottom seal prevents flow through the piston. Preferably, as illustrated, the bottom seal is substantially coplanar with the rib 28 on the piston thereby improving the efficacy of the seal between the piston and barrel at this zone at this time.

When the syringe is filled and ejection is to commence for air venting or for the injection of vaccine or like, the piston rod is moved in the direction of the arrow A Figure 6 relative to the barrel, again in conventional manner. However this causes lost motion between the parts namely the rod and piston to the extent of the clearance 50 Figure 4 which displaces the projections out of the recess 40 Figure 6 and releases them from the confines of the recess. This movement out of the clearance is assisted because displacement of the piston is resisted by the fluid in the barrel. The movement renders the bottom seal ineffective but the top seal between the face 32 and the head 12 becomes effective at the completion of the lost motion. Increased pressure applied to the piston rod for the ejection step increases the sealing pressure on the top seal. Ejection takes place and may be completed.

It will be noted that after completing the ejection stroke, Figure 7, that the piston projections 18 seat on the shoulder 36 but piston 22 does not: Figures 4 and 7 should be compared in this respect. This difference is due to the location of the lost motion gap or clearance.

It is an important advantage of the invention that the use of the syringe is entirely conventional in the sense of the nature of the filling and ejection operations. However, if the user attempts to refill the syringe for a second cycle of operations by merely repeating the conventional filling stroke, that is displacing the piston rod in the direction of arrow B Figure 7 after completion of the previous ejection stroke, the result is that the projections do not enter recess 40. They were released from the confines of the recess 40 at the beginning of the ejection stroke and due to the nature of the synthetic resinous material used for injection moulding, being either floppy or slightly springy, will move out of alignment with the recess, that is (in this embodiment) will separate. The separation of the projections may be only slight but if the transverse spacing of their free ends is too great for them to enter the recess 40 when the piston rod is moved in the refilling direction, the nett result of the operation is to straighten the projections so as to pull them from the nearly doubled positioned into an aligned position and the piston rod then pulls through the piston and separates from the same as shown in Figure 8. Once this has occurred reinsertion and re-connection by any mean is virtually impossible since the piston rests at the bottom of the barrel.

Whilst the illustrated embodiment has a pair of projections, the invention can also be carried out using only a single one, with an appropriately shaped recess 40 to suit. The illustrated version is preferred because it is unnecessary to orientate the piston and piston rod angularly in the assembly step of Figure 1, which is or might be necessary if only a single projection and an asymmetric piston recess were employed.

In the foregoing descriptions one of the seals to prevent flow of the vaccine or like through the piston, is provided by an enlargement or rib on the axially extending stem 14 which extends through the piston. After relative movement between the piston and the extension the enlargement no longer registers with the appropriate portion of the piston bore, so providing a through path for flow of the vaccine or like. However, the same effect can be provided by alternative seal arrangements not using enlargements on the extension 14 or its equivalent. For example the extension may be generally cylindrical and of an appropriate diameter to make sealing engagement with a minimum diameter portion of the piston bore, so as to provide the seal. In this case, in order to provide for flow of a vaccine through the piston, the extension 14 or its equivalent may be provided with grooves or channels in its surface. The grooves or channels will be registered with and extend across the minimum diameter portion of the piston bore after the relative movement in one direction, so as to provide for through flow of a vaccine or like, but will be out of register so as to allow the seal to be established after relative movement in the opposite direction, for example at the time when vaccine is to be drawn into the syringe in the first (and only) cycle of use. The absence of such enlargement on the extension or equivalent may facilitate assembly. This alternative arrangement is not illustrated in the drawings.

## Claims

1. A disposable syringe comprises a barrel, a piston and a piston-rod; in which the piston and rod are connected together by a projection on one of them extending through a bore in the other of them, said projection having a folded over end which is, in one working position, trapped and contained in an enlargement of said bore, and the projection and bore being of such lengths as to allow for lost motion of the one relative to the other, whereby the syringe can only effect a single operational cycle before the said end disengages from the enlargement and frees the end so as to allow the piston and rod to disengage from one another if a further operational cycle is attempted.

2. A disposable syringe as claimed in Claim 1 wherein a pair of parallel symmetrical headed projections are provided on the end of the rod and said bore enlargement is a recess concentric to the piston axis.

3. A disposable syringe as claimed in Claim 2 wherein the said recess is frusto-conical having the smaller end of the frusto-cone adjacent the free end of the piston.

4. A disposable syringe as claimed in Claim 1 wherein a first seal is provided between the piston and piston rod by a peripheral rib on the rod which is locatable within a complimentary annular zone in the piston.

5. A disposable syringe as claimed in Claim 1 wherein a second seal is provided between the piston and piston rod by a frusto-conical end of the piston and an abutting face on the piston rod.

6. A disposable syringe as claimed in Claim 4 and Claim 5 wherein the seals are disposed so that when the pisto and rod are in one position relative to one another one of the seals is effective, and after the lost motion so that the parts are in the other of their possible relative positions, the other of the seals is effective.

7. A disposable syringe as claimed in any of Claims 1-3 wherein a first seal is provided between the piston and piston rode by a portion of the rod which extends through an annular zone in the piston, said first seal being effective after relative movement between said piston and piston rod in one direction, and said piston rod being provided with one or more grooves or channels which register with said piston zone after relative movement in the opposite direction so as to allow for flow through said grooves or channels from one side of the piston to the other.

8. A disposable syringe substantially as described with reference to the drawings.

Fig.1

Fig. 2

Fig. 3

Fig. 4

Fig. 5

Fig. 6

Fig. 7

Fig. 8